# EUROPEAN PATENT SPECIFICATION

(11) **EP 4 199 842 B1**
(45) Date of publication and mention of the grant of the patent: **18.02.2026**
(21) Application number: 21859244.2
(22) Date of filing: 20.08.2021
(51) Int. Cl.: A61B 17/22, A61B 17/00, A61B 17/221, A61M 1/00, A61M 25/00, A61B 17/3207

(54) **DYNAMIC ASPIRATION CATHETER**
DYNAMISCHER ASPIRATIONSKATHETER
CATHÉTER D'ASPIRATION DYNAMIQUE

(30) Priority: 21.08.2020 US 202063068684 P
(43) Date of publication of application: 28.06.2023
(73) Proprietor: MicroVention, Inc., Aliso Viejo, CA 92656 (US)
(72) Inventor: DHOLAKIA, Ronak, Aliso Viejo, California 92656 (US); RANGWALA, Hussain, Aliso Viejo, California 92656 (US)
(74) Representative: Bosch Jehle Patentanwaltsgesellschaft mbH
(86) International application number: PCT/US2021/047008
(87) International publication number: WO 2022/040595

(56) References cited:
- US-A- 5 688 234
- US-A- 5 688 234
- US-A1- 2007 060 888
- US-A1- 2007 060 888
- US-A1- 2007 250 096
- US-A1- 2013 226 146
- US-A1- 2017 252 536
- US-A1- 2018 049 759
- US-B2- 10 213 582
- US-B2- 6 547 779

## Description

### RELATED APPLICATIONS

This application claims benefit of and priority to U.S. Provisional Application Serial No. 63/068,684 filed August 21, 2020, entitled *DYNAMIC ASPIRATION CATHETER AND METHODS.*

### BACKGROUND OF THE INVENTION

Many diseases result from the presence of undesirable material, most notably clots or thrombus, in blood vessels and heart chambers. Clots within vessels can be formed from blood cells, collagen, cholesterol, plaque, fat, calcified plaque, bubbles, arterial tissue, aggregates of proteins (e.g., fibrin), and/or other miscellaneous fragments or combinations thereof. Clots can lodge, for example, in narrow regions of blood vessels that feed the major organs, and therefore, cause loss of oxygen-rich blood flow to surrounding tissues resulting in localized cell death or micro-infarcts. Cerebral micro-infarcts can cause stroke leading to confusion, disturbance of speech, paralysis, visual disturbances, balance disturbances and even death. In the heart, clots can cause myocardial infarcts, i.e., heart attacks. If the clots are left untreated, they are potentially life threatening, and therefore, the presence of clots in the blood vessels need immediate medical intervention.

Clots can typically be treated or eliminated by utilizing biologic intervention, surgical intervention, or a combination of the two. Biologic treatments involve the delivery of agents directly to the clots by a catheter to either dissolve or at least stabilize it until the body can eliminate it. However, the disadvantage of treating clots with biologic agents is that they expose much of the body to the agents, potentially leading to life-threatening bleeding complications.

Alternately or additionally, mechanical means can be used to remove the clots from a patient's vessel. Mechanical treatments typically involve aspiration, maceration, and compression of the clots within the vessel and finally removing it either by invasive surgery or by non-invasive means, such as with an aspiration catheter connected to an aspiration source (e.g., pump or syringe). The distinct advantage of invasive or non-invasive mechanical treatment is that it directly attacks the clots and eliminates the vascular obstruction without affecting non-diseased areas of the body, unlike biologic agents.

The mechanical, non-invasive clot removal treatments generally relate to thrombectomy procedures, more particularly, peripheral thrombectomy procedures and venous thrombectomy procedures, such as deep venous thrombosis (DVT) treatment or intracranial distal aspiration.

Acute ischemic stroke treatment using vascular aspiration embolectomy involves aspiration techniques to draw clots into a catheter via suction and has been proven to be as effective as mechanical thrombectomy using stent retrievers which deploy self-expanding stent-like capture devices to capture and removed the thrombus. Aspiration embolectomy has gained favor with a major section of the neuro-interventional physicians due to the ease of procedure and its cost effectiveness.

In aspiration embolectomy, a distal aspiration catheter (DAC) is used to remove the clot or emboli from the vessel. At the first step of the procedure, a distal end of the catheter is positioned adjacent to the clot and then a vacuum force is applied through a lumen in the catheter using a pump or large volume syringe. The suction force used by the syringe or pump partially ingests or pulls in the emboli into the distal portion of the DAC. In such instances, when the emboli are partially ingested, repeated passes of the device may be needed to recanalize the vessel.

Successful clinical outcome of the patients undergoing aspiration embolectomy, however, depends on the "first-pass effect". The first-pass effect is defined as the complete or near-complete recanalization of the affected vessel after one pass of an embolectomy device by complete removal of the emboli in the first attempt.

Inside the vessels, however, blood clots can undergo a process called organization where the soft gel-like red/purple clot is transformed into a firmer, whitish clot by the cross-linking of proteins such as fibrin. Clots may also form firm masses with the forward pressure of blood flow over time. Firm clots can form a large mass of well embedded, mature clots over time which are less flexible and can be difficult to completely ingest into the traditional DAC when suction is applied with the attached pump or aspiration syringe kit. This partial ingestion of thrombus/emboli using a traditional DAC, therefore, reduces the chances of a first pass effect and requires additional passes of the traditional DAC for complete removal of thrombus/emboli. Additionally, there is also the possibility of fragmentation of the well embedded, mature thrombus/emboli during removal which can travel to other areas in the blood vessels. The above risk factors for removing a large mass of well embedded, mature clots by using a traditional DAC negatively impacts the clinical outcome of a patient by reducing the chance of a "first pass effect".

Furthermore, the use of a stent retriever in combination with a traditional DAC, known as the Solumbra technique, has become a popular approach to mechanical thrombectomy for acute ischemic stroke. However, the Solumbra technique has not been shown to improve the first pass effect over aspiration embolectomy alone.

Hence, there is a need for an advanced DAC which can at least overcome the problems discussed above for use of a traditional DAC, particularly for removing well embedded, mature clots and improving the clinical outcome in patients by complete recanalization of the vessel after one pass of the DAC device.

US 10 213 582 B2 describes a system of devices for treating an artery wherein the system includes an arterial access sheath adapted to introduce an interventional catheter into an artery and an elongated dilator positionable within the internal lumen of the sheath body. The system also includes a catheter formed of an elongated catheter body sized and shaped to be introduced via a carotid artery access site into a common carotid artery through the internal lumen of the arterial access sheath. Also disclosed is a catheter including a structure for morcelating a thrombotic occlusion as it is being aspirated into the catheter.

US 2007/250096A1 describes a catheter including a flexible elongate catheter tube having a distal end, a proximal end, and a lumen defined therein, the proximal end being connectable to a suction device for evacuating the lumen, and a thrombus cutter disposed in the catheter tube near the distal end thereof, the thrombus cutter including at least one cutting edge directed radially inward from an inner wall surface of the lumen.

US 2007/060888 A1 describes an aspiration catheter comprising a catheter body having an aspiration lumen therethrough, wherein the distal opening of the aspiration lumen can be modified in various ways or can have the addition of structure in order to disrupt sealing between the port and material being aspirated.

US 5 688 234 A describes an apparatus for the treatment of a thrombotic occlusion in a vessel of a patient comprising a flexible elongate tubular sheath having proximal and distal extremities.

### SUMMARY OF THE INVENTION

The present invention is directed to a clot removal catheter which can be used to fatigue a clot so that it can be completely removed from a blood vessel. These devices may be particularly helpful for firm and/or well-embedded clots that might otherwise present difficulties during a removal process. In some instances, these embodiments can lead to recanalization of the blood vessel after one pass of the catheter.

According to the present invention, there is provided a clot removal catheter as defined by independent claim 1. Further advantageous features of the present invention are defined by the dependent claims.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which:
Fig. 1 illustrates a perspective view of an aspiration embolectomy as a treatment for acute ischemic stroke using a traditional DAC in which the clot is partially ingested after application of a suction force.
Fig. 2 illustrates a perspective view of a DAC with two mandrels in one embodiment of the present invention.
Fig. 3 illustrates a perspective view of an aspiration embolectomy as a treatment for acute ischemic stroke using the DAC having two mandrels in one embodiment of the present invention.
Fig. 4 illustrates a perspective view of an aspiration embolectomy as a treatment for acute ischemic stroke using the DAC having two mandrels in one embodiment of the present invention.
Fig. 5A illustrates a perspective view of a handle of a DAC and attached mandrels in one embodiment of the present invention.
Fig. 5B illustrates a perspective view of a handle of a DAC and attached mandrels in one embodiment of the present invention.
Fig. 6 illustrates a perspective view of the DAC attached with the handle shown in Figs. 5A and 5B in one embodiment of the present invention.
Fig. 7A illustrates a perspective view of a mandrel having a cylindrical wedge- shaped distal portion.
Fig. 7B illustrates a perspective view of a mandrel having a flat wire like distal portion.
Fig. 7C illustrates a perspective view of a mandrel having a half cylindrical distal portion.
Fig. 8 illustrates a perspective view of a DAC with four mandrels in one embodiment of the present invention.
Fig. 9 illustrates a perspective view of an aspiration embolectomy as a treatment for acute ischemic stroke using the DAC having plurality of cutting blades in one embodiment of the present disclosure.

### DESCRIPTION OF EMBODIMENTS

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

In this specification the non-SI units 'inch' and 'French' are used, which may be converted to the SI or metric unit according to the following conversion: 1 inch ≈ 25.4 mm and 1 French ≈ 0.333 mm.

The terms clot, thrombus, and embolus are used interchangeably within this specification. Generally, these terms mean a mass or connected group of blood cells, collagen, cholesterol, plaque, fat, calcified plaque, bubbles, arterial tissue, aggregates of proteins (e.g., fibrin), and/or other miscellaneous fragments or combinations thereof.

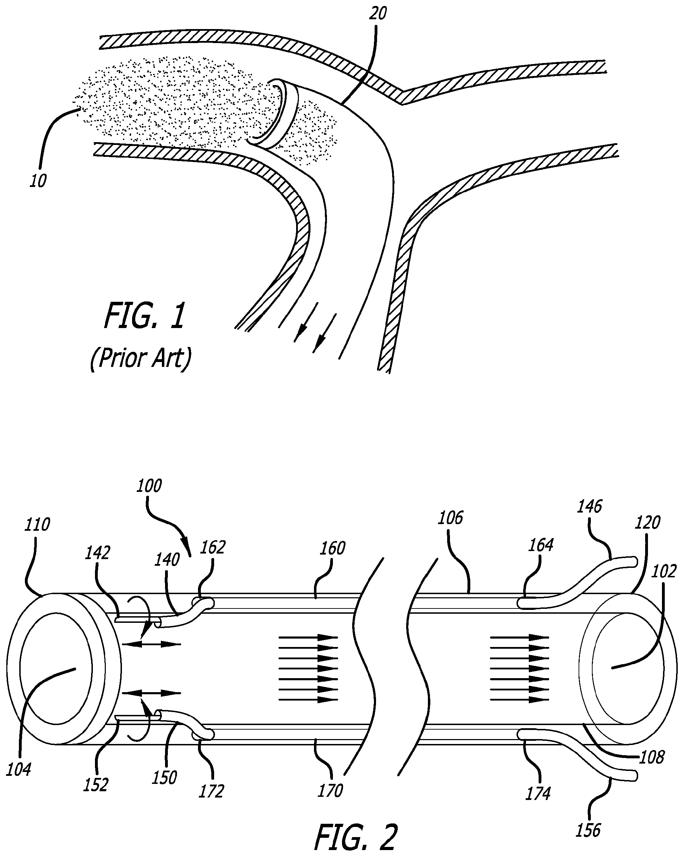

As previously discussed, existing aspiration catheter techniques may not be effective in removing large amounts of firmly embedded clot from inside a blood vessel after one pass of the aspiration catheter because of a lack of complete ingestion of the clot inside the aspiration catheter and/or fragmentation of the clots during removal. Fig. 1 illustrates a traditional distal access catheter (DAC) 20 that is used to remove a large amount of firmly embedded clot 10 from inside the vessel. The DAC 20 might typically have an inner diameter profile ranging from 1.3 (4F) to 4 (12F) mm. In one example, the clot 10 is lodged in a middle cerebral artery causing large vessel occlusion acute ischemic stroke. During the removal procedure, the distal end of the DAC 20 is positioned adjacent to the clot 10 and the clot is partially ingested or suctioned inside a lumen of the distal portion of the DAC 20 after a vacuum force is applied at a proximal end of the lumen with an attached pump or aspiration syringe.

If the clot 10 is firmly embedded and larger than the diameter of the DAC 20, applying vacuum force alone may not be effective in recanalization of the vessel. In other words, complete removal of the clot after a first pass of the device may not be possible. While it may be possible to use a larger diameter DAC and/or apply a stronger vacuum force, a larger profile DAC can be more challenging to navigate to achieve distal intracranial circulation and may be detrimental to the vessel lumen, causing dissection/vasospasm.

The present invention seeks to address the deficiencies of the existing devices and techniques by removing a clot after one pass of a DAC during a procedure. Some of the embodiments described herein are directed to a DAC (also referred to more generally as a catheter) that includes a mechanism for causing clot fatigue. More specifically, a DAC includes a mechanism for creating physical stress to a clot in a way that reduces the hardness, size, and/or cohesion of the clot. This stress can be achieved with mandrels, cutting devices with sharp and/or dull blades, or similar components that are configured to move relative to the clot.

The term "clot fatigue" is generally defined as a process to apply mechanical stress to a clot to alter/reduce stiffness, size, and/or cohesion to cause the clot to partially or completely break down. Performing procedures to fatigue a clot can be particularly helpful for aspirating a clot into a catheter and removal from a patient.

The DAC can be used to remove clots/emboli from any kind of blood vessels. Vessels from which the clot/emboli may be removed, in accordance with an embodiment of the present invention, include acute ischemic stroke, those within the pulmonary circulation (e.g., pulmonary arteries), systemic venous circulation (e.g., jugular vein, sigmoid sinus, transverse sinus, superior and inferior sagittal sinus, vena cava, pelvic veins, femoral veins, and subclavian veins), or arterial circulation (e.g., aorta or its large and medium branches).

Some embodiments of the DAC of the present invention comprises a plurality of protruding structures, for example, mandrels, at a distal portion in a lumen of the DAC. Upon engaging the clot with the distal end of the DAC, the distal portions of the protruding structures can undergo rotational and/or translational motion to induce "clot fatigue" which helps the clot to be partially or completely ingested inside the lumen of the DAC by the application of a vacuum force.

Fig. 2 illustrates one embodiment of a DAC 100 that includes one or more mandrels configured to cause mechanical stress to the clot and thereby induce clot fatigue. The DAC 100 is illustrated with a first mandrel 140 with a distal portion 142 and a second mandrel 150 with a distal portion 152, though any number of mandrels are possible (e.g., 2, 3, 4, 5, 6, 7, or 8). The mandrels 140 and 150 can be configured to move in a variety of different ways and angles to create or induce fatigue or stress on a clot. For example, the mandrels can move longitudinally (i.e., axially relative to the catheter body), rotationally (i.e., rotating around an axis of the mandrel), radially inwards (i.e., towards a cross sectional center of the catheter), radially outwards, (i.e., away from a cross sectional center of the catheter), side-to-side (i.e., towards and away from an outer circumference of the catheter), or any combination thereof.

The mandrels 140, 150 can be engaged or activated upon engagement with the clot. Depending on the positioning and configuration of the mandrels 140, 150, this may involve initially applying aspiration to the catheter 100 to at least partially draw the clot near the distal end of the DAC 100 and preferably within the aspiration lumen 101 of the DAC 100. However, it is possible that the mandrels 140, 150 can be configured to fatigue or stress the clot without the need for initial aspiration, such as by being configured to extend to a very distal edge of the DAC 100 or even slightly beyond the distal end of the DAC 100.

Generally, the mandrels described in this specification are bodies having an elongated shape with a length sufficient to extend between a proximal portion and a distal portion of the DAC 100. The mandrels preferably have at least some rigidity sufficient to allow distal and proximal movement through passages in the DAC 100. For example, the mandrels may be composed of wire alone, coated wire, a helical wire coil, or similar arrangements. The mandrels may be generally similar to known guidewire designs, and in some circumstances, guidewires can be used as mandrels.

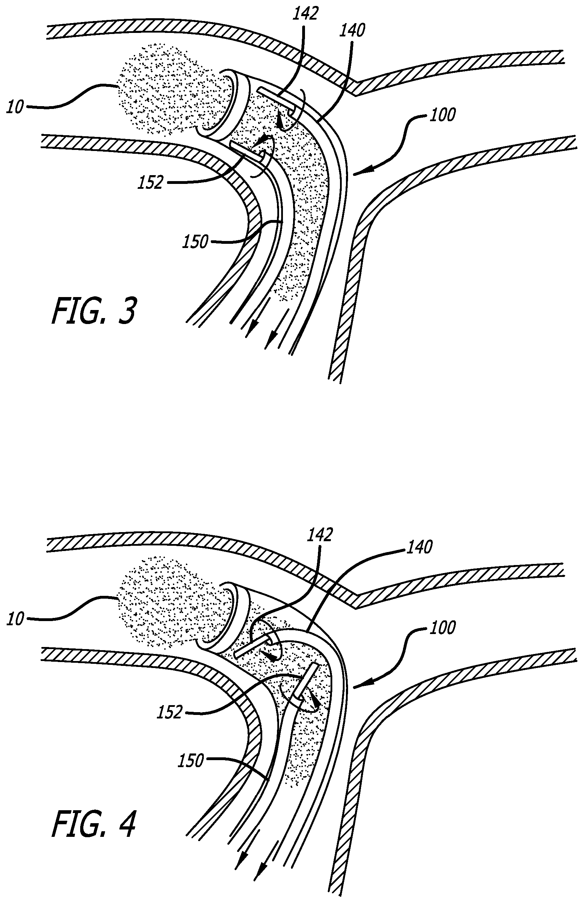

As illustrated in Fig. 3, the fatigued clot has an overall reduced diameter, reduced hardness, and/or reduced cohesion that allows it to be better ingested completely inside the DAC 100, preferably after one pass of the instrument. Hence, increased suction force may not be necessary, which may otherwise cause blood loss to other organs and shock in a patient undergoing the treatment.

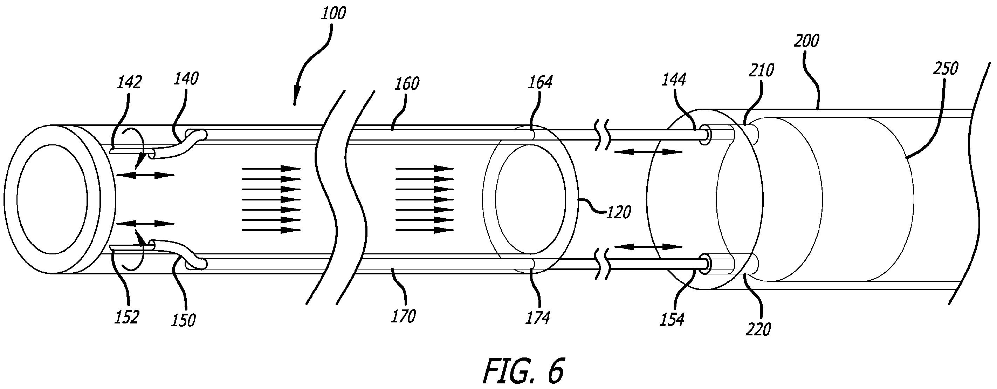

The DAC 100 may include a tubular catheter body having a stiff proximal shaft. The stiffness of the proximal shaft helps to attach the proximal end 120 of the DAC 100 with a handle 200 (Figs. 6A and 6B) or catheter hub. The stiffness of the tubular catheter gradually reduces from the proximal to distal portion. A distal end 110 of the DAC 100 comprises a soft tip which ensures smooth navigation of the DAC 100 to the distal sections of the intracranial blood vessels.

In some embodiments, the DAC 100 comprises a lumen 101 having a proximal section 102 and a distal section 104. The lumen 101 may have a range of different sizes, depending on the location of use within a patient, such as a diameter in a range of about 6-8 French.

In some embodiments, a hydrophilic coating is disposed on an outer portion of the distal section 104 of the DAC lumen. The hydrophilic coating is used to reduce friction with the vessel lumen during navigation of the DAC 100 inside the distal sections of the intracranial blood vessels. In one example, about 60 centimeters of the distal portion of the DAC lumen 104 comprises hydrophilic coating to reduce friction with the vessel lumen during the navigation. In another example, about 30 centimeters of the distal portion of the DAC lumen 104 comprises a hydrophilic coating to reduce friction with the vessel lumen during the navigation. In yet another example, about 20 centimeters of the distal portion of the DAC lumen 104 comprises a hydrophilic coating to reduce friction with the vessel lumen during the navigation. In some embodiments, the hydrophilic coating comprises but is not limited to commercial coating solutions such as Hydak, which is a curable UV and heat treatment (Biocoat, Horsham, PA), or Lubricent, which is a curable UV treatment (Harland Medical Systems, Eden Prairie, MN).

The DAC 100 also includes a passage or channel for each mandrel that extends between a proximal end of the DAC 100 and a distal end of the DAC 100. These passages or channels generally have a distal opening through which the mandrel can be positioned out of and a proximal opening through which the mandrel can extend from (e.g., for manual actuation or into a motorized handle for motorized actuation). In some embodiments, each of the channels are configured to carry one mandrel, though larger diameter channels configured to carry multiple mandrels are also possible. Channels and their distal openings can be positioned at different circumferential locations relative to each other. For example, two channels may be located at about 180 degrees relative to each other (i.e., opposite diametric positions), 90 degrees relative to each other, 45 degrees relative to each other, 25 degrees relative to each other, or any angle and/or angle combination.

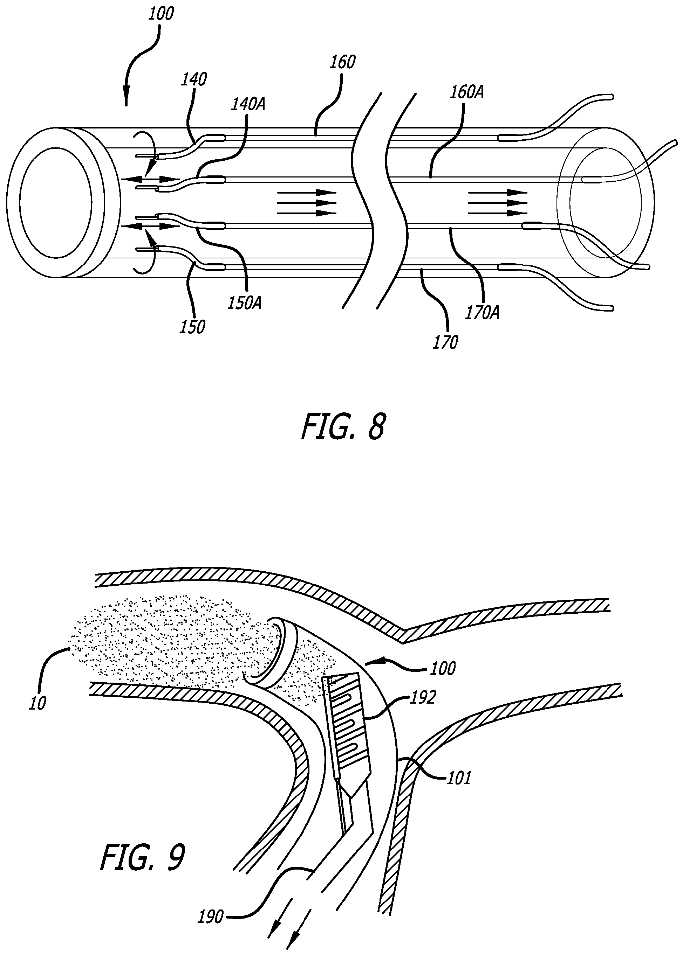

The positions of the channels along the periphery I of the DAC 100 (e.g., within the wall or between wall layers of the DAC 100) may have several advantages. First, this arrangement can accommodate a relatively large number of channels while maintaining a sufficient size of the aspiration lumen 101. In one example, Fig. 2 illustrates that two luminal channels 160 and 170 are located along the periphery of the DAC 100 and a first mandrel 140 is positioned inside luminal channel 160 and a second mandrel 150 is positioned inside luminal channel 170. In another example, Fig. 8 illustrates that four luminal channels 160, 160A, 170, 170A and four mandrels 140, 140A, 150, 150A are located along the periphery of the DAC 100. However, it should be understood that other numbers of channels and mandrels can be used, such as 2, 3, 4, 5, 6, 7, 8, or more channels and mandrels.

Another advantage of the peripheral positions of the luminal channels and mandrels is that the mandrels do not obstruct the passage of the thrombus inside the lumen 104 of the DAC 100 and therefore increases the chance of the first pass effect. While placing one or more mandrels through the aspiration lumen 101 is possible according to the present invention, their potential blockage of the clot makes the use of the described channels preferable.

Fig. 2 illustrates one specific arrangement of passages or channels located between an outer cylindrical jacket 106 and an inner cylindrical liner 108. A first luminal channel 160 and a second luminal channel 170 are positioned inside a space between the outer cylindrical jacket 106 and inner cylindrical liner 108. The first luminal channel 160 may comprises a first port 162 at the distal portion of the DAC 100 and a second port 164 at the proximal portion of the DAC 100. A first mandrel 140 is positioned within a lumen of the first luminal channel 160 so that its distal portion 142 extends out the distal first port 162 and its proximal end 146 extends out of the proximal second port 164.

Similarly, the second luminal channel 170 comprises a first port 172 at the distal portion of the DAC 100 and a second port 174 at the proximal portion of the DAC 100. A second mandrel 150 is positioned within a lumen of the second luminal channel 150 so that its distal portion 152 extends out of the distal first port 172 and its proximal end 156 extends out of the proximal second port 174.

The proximal second ports 164, 174 can be positioned in a proximal location opening on the outer surface of the DAC 100 so that they can be used similar to a rapid exchange port and allow for manual actuation by a user. Alternately, the proximal second ports 164, 174 can open within a catheter hub in a manner that allows manual actuation by the user. Alternately, the proximal portions may open directly into a handle to allow for motorized actuation, as discussed with regard to further embodiments later in this specification.

Although, two channels 160 and 170 are illustrated in Fig. 2, any number of luminal channels and mandrels are possible. For example, 1, 2, 3, 4, 5, 6, 7, 8, 9, or 10 luminal channels may be included. For example, the alternate embodiment of Fig. 8 illustrates four luminal channels 160, 160A, 170, 170A and four mandrels 140, 140A, 150, 150A along the periphery of the luminal segment of the DAC 100.

The distal first ports and/or the distal portions of the mandrels (e.g., first ports 162, 172, distal portions 142, 152, and mandrels 140, 150) can be configured to direct the distal portion of the mandrels into the main aspiration lumen 101 of the DAC 100 at different angles relative to the axis of the DAC 100. For example, the distal portion of a mandrel can be configured to exit the first port in an angle generally parallel to the axis of the DAC 100 (e.g., as shown in Fig. 2), or at an angle relative to the axis of the DAC 100 of 5, 10, 15, 20, 25, 35, 40, 45, 50, 55, 60, 65, 70, 75, 80, 85, 90, or any angle between or beyond those numbers. Additionally, the distal first ports (e.g., first ports 162, 172) can either be located at the same distance from the distal end of the catheter 100 or at different distances. In other words, each of the distal first ports can be located at different longitudinal locations. In the case of more than two distal first ports, some ports may be located at the same longitudinal position and other at a different position (e.g., two ports at 2 mm and two ports at 4 mm from a distal end of the catheter 100).

The angle of exit from the first port can be achieved in several different way. First, the first port itself can be curved to direct the mandrel out at a specific angle. Second, at least the distal portion of the mandrel can be composed of a shape memory material that has a memory shape or heat-set shape that causes it to bend when unconstrained. Either or both of these techniques can be used.

In some embodiments of the present invention, to ensure "clot fatigue", the mandrels can be moved translationally (i.e., proximally and distally), rotationally relative to an axis of the mandrel, or a combination of the two. Additionally, multiple mandrels may have the same movement characteristics or different movement characteristic. For example, one mandrel may rotate while another mandrel moves translationally. In another example, one mandrel may distally extend and remain stationary while the other mandrel moves translationally.

In one example, the translational movement may consist of a mandrel moving proximally and distally by about 0.5 to 5 mm. In another example, the translational movement can cycle back and forth relatively slowly (e.g., about once every 1 to 2 seconds) or relatively quickly (e.g., about once every 0.5 to 0.01 seconds). In another example, the rotational movement of a mandrel can continuously rotate in a single direction or can rotate back and forth, switching directions periodically.

Returning to the DAC 100 example of Fig. 2, both the distal portion 142 of the first mandrel 140 and the distal portion 152 of the second mandrel 150 undergo dynamic movement (e.g., rotational and translational) inside the partially ingested clot 10 to macerate it, as can be seen in Fig. 3. The macerated clot 10 has an overall weakened or decreased structure which allows it to be ingested completely or nearly completely inside the DAC 100 when aspiration is applied within the lumen of the DAC 100.

In one specific example technique shown in Fig. 4, "clot fatigue" can be performed when the distal portion 152 of the second mandrel 150 remains distally extended to hold the partially ingested clot 10 in place while the distal portion 142 of the first mandrel 140 provides the dynamic rotational and translational motions and/or longitudinal oscillation. In yet some other embodiments, the distal portion 142 of the first mandrel 140 may hold the partially ingested clot 10 in place and the distal portion 152 of the second mandrel 150 may provide the dynamic rotational and translational motions and/or longitudinal oscillation in the clot to enable "clot fatigue". The fatigued clot 10 has overall weakened structure and/or reduced diameter which allows it to be ingested completely or nearly completely inside the lumen of the DAC 100 when aspiration is applied.

The distal portions (e.g.,142 and 152) of the mandrels (e.g.,140 and 150) described in this specification can have a variety of different shapes to help penetrate or break up the structure of a clot. For example, the mandrel ends may form a point, a flattened rectangular shape, a triangular shape, a partial cylindrical shape, or similar variations.

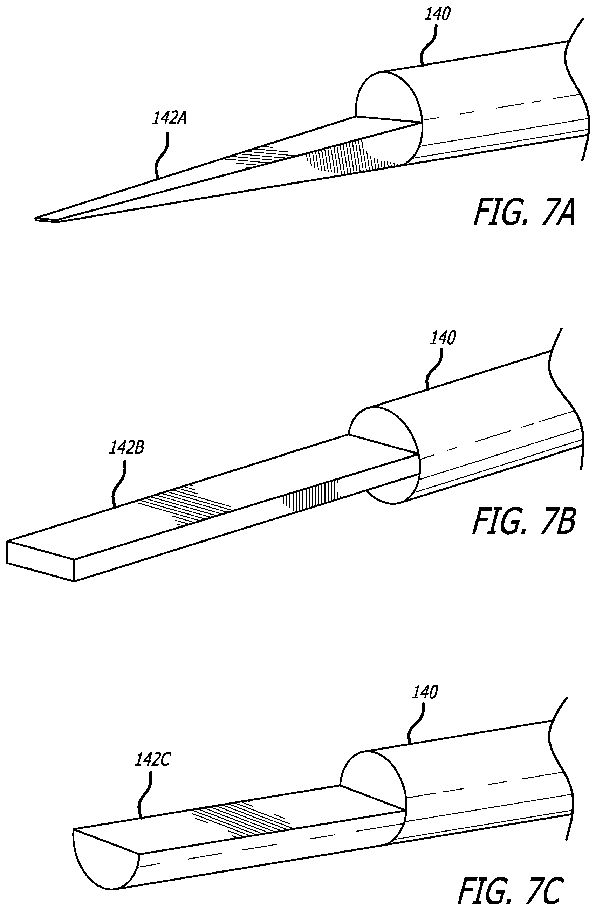

Fig. 7A illustrates one specific example shape 142A which decreases in diameter in a distal direction, has a flat top surface and a rounded lower surface. Fig. 7B illustrates another specific example shape 142B which forms a rectangular shape with a flat upper and lower surface. Fig. 7C illustrates another specific example of a half cylinder shape 142C, having a flat upper surface. In some embodiments, the diameters of the shapes or structures of the distal portion 142 of the first mandrel 140 and the distal portion 152 of the second mandrel 150 depend on the internal diameter of the lumen 101 of the DAC 100. In one specific example, the diameter of the distal portion 142 of the first mandrel 140 and the distal portion 152 of the second mandrel 150 may be about 0.0014 inches, when the DAC 100 is used to remove clots from the blood vessels inside the brain.

In some embodiments of the present invention, the mandrels (e.g., 140 and 150) may be made of shape memory materials e.g., nitinol, stainless steel, or cobalt-chromium. Additionally or alternatively, the mandrels may be made of radiopaque materials, for example, platinum or tantalum. In some embodiments, the mandrels 140 and 150 have guidewire like flexibility so that they can be easily navigated through the first luminal channel 160 and the second luminal channel 170 of the DAC 100.

While the mandrels described in this specification can be custom made for the DAC 100, preexisting guidewires can also be used (e.g., a 0.035 inch guidewire).

Additionally, one of the channels 160, 170 can initially be used with a guidewire to initially position the DAC 100 at a desired clot location. For example, a guidewire can be initially positioned so that its distal end is near or within a target clot. A distal end of the guidewire can be placed into the distal first port 162 and into channel 160 until it extends out the second port 164. The DAC 100 can then be advanced over the guidewire until its distal end is located near the clot. Finally, the guidewire can be withdrawn and a mandrel 140 can be advanced into the channel 160, allowing the clot fatiguing and aspiration procedure to proceed accordingly.

As previously discussed, the translational and rotational motions of the mandrels can be performed manually in some embodiments. For example, a physician may grasp a proximal end of one or more mandrels and move them proximally/distally, as well as rotationally.

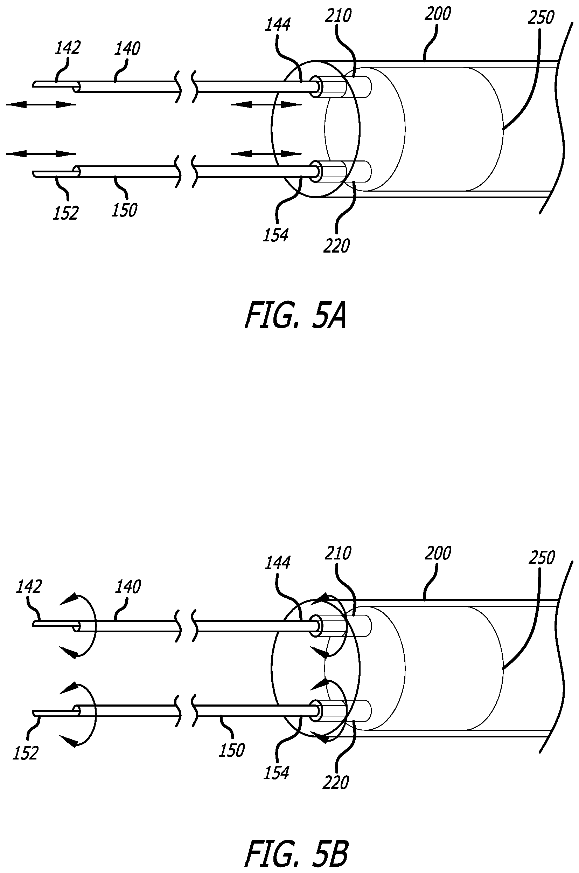

In other embodiments, the mandrels 140 and 150 may be moved via a motorized mechanism that converts the movement of an electric motor into the desired translational and rotational movement. As illustrated in the embodiment of Figs. 5A and 5B, the proximal end 144 of the mandrel 140 is attached to a first interface 210 and the proximal end 154 of the mandrel 150 is attached to a second interface 220. The proximal end of the first interface 210 is attached to a motorized actuator 250 and the proximal end of the second interface 220 is also attached to the motorized actuator 250. The first interface 210, second interface 220, and actuator 250 are placed within a handle 200 and is activated by an interface element, for example, by pressing a button (not shown in the drawings) positioned on the handle 200 and electrically connected to the actuator 250. Fig. 5A demonstrates translational motion of the mandrels 140 and 150 and Fig. 5B demonstrates rotational motion of the mandrels 140 and 150. Both movements can be used simultaneously or at different times on the same mandrel.

Fig. 6 illustrates the DAC 100, as a whole, with the attached motorized handle 200. In this embodiment, the port 164 from which the mandrel 140 enters inside the first luminal channel 160 is positioned at the proximal end 120 of the DAC 100. Similarly, the port 174 from which the mandrel 150 enters inside the second luminal channel 170 is positioned at the proximal end 120 of the DAC 100. Hence, the mandrel 140 is pushed through the port 164 and exits through port 162, and the mandrel 150 is pushed through the port 174 and exits through port 172 such that the distal portion 142 of the first mandrel 140 and the distal portion 152 of the second mandrel 150 are positioned inside the distal portion 104 of the DAC lumen. The handle 200 can be separately attachable to the proximal end of the DAC 100 or can be integrally formed with the DAC 100. In some embodiments, when the actuator 250 is activated, for example, by pressing a button on the handle 200, the distal portion 142 of the first mandrel 140 and the distal portion 152 of the second mandrel 150 undergo translational and rotational motions to induce "clot fatigue" in the partially ingested clot inside the distal portion 104 of the DAC lumen.

In some embodiments of the present invention, the distal portion 142 of the first mandrel 140 and the distal portion 152 of the second mandrel 150 do not protrude outside the distal end 110 of the DAC. For example, a mechanism to prevent movement outside of the DAC can be included, such as a hard stop or lip (not shown in the drawings) at the distal end 110 of the DAC 100 to prevent further distal movements of the distal portion 142 of the first mandrel 140 and the distal portion 152 of the second mandrel 150 outside the catheter lumen and into the blood vessels.

In an alternate embodiment, 'clot fatigue" can be created by reducing the size of the clot by using one or more cutting elements located within a distal portion of the main lumen 101 of the DAC 100. The one or more cutting elements are generally defined as a structure comprising one or more cutting surfaces having a sharp, partially sharp, and/or dull surface that are sized, positioned, and/or configured to at least partially cut into a clot.

The cutting element can have a variety of different shapes, such as a flat planar shape, a point (e.g., surface 142A of Fig. 7A may be considered a cutting element), a cylindrical shape, crossing planar shapes (e.g., X shape), a helical corkscrew shape, or similar designs. In some embodiments, the edges of the cutting blades may be sharp, dull, or a combination of both (e.g., sharp regions and dull regions). In a specific example, the cutting element can be dull on the outside of the cutting element but have sharp cutting surfaces on the inside of the cutting element such that they may act to pull in the clot and create cuts within the cutting element (e.g., a helical shape with sharp inner surfaces).

In some embodiments, the cutting element may be attached to or even integral with a distal end of a mandrel or similar structure so that it can be manually actuated in a manner similar to that described for the other mandrels in this specification. In other embodiments, the cutting element may be connected to a shaft or other elongated element that is configured for manual or motorized rotation. In other embodiments, the one or more cutting elements may be stationary by being fixed to an inner surface or other component within the lumen of the DAC 100 (e.g., a helical shape with an inner sharpened edge.

Figure. 9 illustrates one embodiment of a DAC 100 with a generally tubular shaped cutting element 192 that forms a plurality of cutting surfaces. In this embodiment, the cutting element 192 is connected to an elongated shaft 190 and configured to rotate either manually or in a motorized manner. For example, an actuator or similar motorized device can be connected to a proximal end of the elongated shaft 190 (not shown in the drawings) and can be activated electrically by pressing a button (not shown in the drawings). The cutting element 192 can be moved translationally (i.e., proximally and distally), rotationally relative to an axis of the cutter 190, or a combination of the two. The cutting element 192 comprises a plurality of sharpened cutting surfaces angled radially or diametrically inward of the cutting element's generally cylindrical shape. This allows the cutting element 192 to draw in the clot while also inducing clot fatigue to the clot.

One method of removing a large and/or well embedded clot is described below in connection with the previously described devices. A guidewire and/or delivery sheath may be initially deployed in the patient such that the guidewire or sheath allows a distal end 104 of the DAC 100 to be positioned near a clot. For example, the DAC 100 may be inserted into the targeted blood vessel over the guidewire and advanced towards the clot such that the distal end 110 of the DAC 100 is positioned against or near the surface of the clot 10.

Once the clot is positioned against or engaged with the distal end 110 of the DAC 100, an aspiration source connected to the DAC 100 is activated to at least partially suck the clot into the distal section 104 of the DAC lumen. Aspiration can be provided by a manually actuated syringe or motorized pump.

Next, "clot fatigue" is induced in the partially ingested clot either by manually operating the mandrels or by actuating an actuator such that the distal ends of the mandrels undergo translational and/or rotational motions into the clot (e.g., Figs. 3, 4). The motion of these mandrels can occur simultaneously with the aspiration or the aspiration can be momentarily stopped during mandrel movement. In either case, aspiration and mandrel movement is continued until the clot moves into the lumen of the DAC 100, at which point the aspiration and mandrel movement can be optionally stopped. At the end of the procedure, the DAC 100 with the clot is withdrawn from the blood vessel. In some embodiments of the present invention, a contrast agent may be injected during the treatment to determine if the recanalization of the blood vessel was successful.

Although the invention has been described in terms of particular embodiments and applications, one of ordinary skill in the art, in light of this teaching, can generate additional embodiments and modifications without departing from the scope of the claimed invention. Accordingly, it is to be understood that the drawings and descriptions herein are offered by way of example to facilitate comprehension of the invention and should not be construed to limit the scope thereof.

The invention is defined by the following claims.

## Claims

1. A clot removal catheter, comprising:
a catheter body (106) having an aspiration lumen (101) opening at a distal end (110) of the catheter body; **characterised by** a first mandrel lumen (160) extending through the catheter body and having a first distal opening (162) within a distal region of the aspiration lumen; wherein the first mandrel lumen (160) is positioned at a periphery of the catheter body; a first mandrel (140) located within the first mandrel lumen; wherein the first mandrel lumen directs a distal end of the first mandrel (140) into the distal region of the aspiration lumen to fatigue a clot that is partially pulled into the aspiration lumen

2. The clot removal catheter of claim 1, further comprising a second mandrel lumen (170) positioned at the periphery of the catheter body (106) and having a second distal opening (172) within the distal region (104) of the aspiration lumen (101); and a second mandrel (150) located within the second mandrel lumen (170); wherein the second mandrel lumen (170) directs a distal end (152) of the second mandrel (150) into the distal region (104) of the aspiration lumen (101) to fatigue the clot (10) that is partially pulled into the aspiration lumen (101).

3. The clot removal catheter of claim 2, further comprising a third mandrel lumen (160A) and a fourth mandrel lumen (170A), both of which positioned at the periphery of the catheter body (106) and having openings within the distal region (104) of the aspiration lumen (101); a third mandrel (140A) located within the third mandrel lumen (160A); and, a fourth mandrel (150A) located within the fourth mandrel (170A) lumen; wherein the third mandrel lumen and the fourth mandrel lumen direct distal ends of the third mandrel (140A) and the fourth mandrel (150A) into the distal region (104) of the aspiration lumen (1101).

4. The clot removal catheter of claim 2, wherein the first mandrel lumen (160) and the second mandrel lumen (170) are positioned within a wall of the of the catheter body (106).

5. The clot removal catheter of claim 4, wherein the first mandrel lumen (160) and the second mandrel lumen (170) are positioned between an outer jacket (106) and an inner liner (170) of the catheter body (106).

6. The clot removal catheter of claim 2, wherein the first mandrel lumen (160) and the second mandrel lumen (170) are positioned at different longitudinal positions from each other, relative to a distal end of the catheter body (106).

7. The clot removal catheter of claim 1, wherein the first mandrel lumen (160) has an angle relative to a longitudinal axis of the catheter body (106) in a range of about 25 degrees to 180 degrees.

8. The clot removal catheter of claim 1, wherein the first mandrel (140) comprises a shape memory material with a memory shape imparted to it to cause a distal portion of the first mandrel (140) to bias radially inwardly into the aspiration lumen (101).

9. The clot removal catheter of claim 1, further comprising a handle portion (200) connected at a proximal end (120) of the catheter body (106); the handle (200) comprising an actuator (250) connected to the first mandrel (140); wherein the actuator (250) moves the first mandrel (140) translationally, rotationally, or both.

10. The clot removal catheter of claim 1, further comprising a stop configured to prevent a distal end (142) of the first mandrel (140) from passing distally out of the aspiration lumen (101).

11. The clot removal catheter of claim 1, wherein the distal portion (142) of the first mandrel (140) comprises a shape of a flattened rectangle, a triangle, or a half cylinder.

12. The clot removal catheter of claim 1, wherein the first mandrel lumen (160) includes a first proximal opening (164) within a proximal portion (120) of the catheter body (106).

13. The clot removal catheter of claim 1, wherein the first distal opening (162) is shaped so that the first mandrel (140) exits at an angle generally parallel relative to a longitudinal axis of the catheter body (106) or a range about 5 degrees to 90 degrees relative to the longitudinal axis of the catheter body (106).

## Patentansprüche

1. Katheter zum Entfernen von Blutgerinnseln, umfassend:
einen Katheterkörper (106) mit einem Aspirationslumen (101), das sich an einem distalen Ende (110) des Katheterkörpers öffnet; **gekennzeichnet durch** ein erstes Dornlumen (160), das sich durch den Katheterkörper erstreckt und eine erste distale Öffnung (162) innerhalb einer distalen Region des Aspirationslumens aufweist; wobei das erste Dornlumen (160) an einem Umfang des Katheterkörpers positioniert ist; einen ersten Dorn (140), der sich innerhalb des ersten Dornlumens befindet; wobei das erste Dornlumen ein distales Ende des ersten Dorns (140) in der distalen Region des Aspirationslumens leitet, um ein Blutgerinnsel, das teilweise in das Aspirationslumen gezogen wird, zu zermürben.

2. Katheter zur Entfernung von Blutgerinnseln nach Anspruch 1, ferner umfassend ein zweites Dornlumen (170), das am Umfang des Katheterkörpers (106) positioniert ist und eine zweite distale Öffnung (172) innerhalb der distalen Region (104) des Aspirationslumens (101) aufweist; und einen zweiten Dorn (150), der sich innerhalb des zweiten Dornlumens (170) befindet; wobei das zweite Dornlumen (170) ein distales Ende (152) des zweiten Dorns (150) in die distale Region (104) des Aspirationslumens (101) leitet, um das Blutgerinnsel (10), das teilweise in das Aspirationslumen (101) gezogen wurde, zu zermürben.

3. Katheter zur Entfernung von Blutgerinnseln nach Anspruch 2, ferner umfassend ein drittes Dornlumen (160A) und ein viertes Dornlumen (170A), die beide am Umfang des Katheterkörpers (106) positioniert sind und Öffnungen innerhalb der distalen Region (104) des Aspirationslumens (101) aufweisen; einen dritten Dorn (140A), der sich innerhalb des dritten Dornlumens (160A) befindet; und einen vierten Dorn (150A), der sich innerhalb des vierten Dornlumens (170A) befindet; wobei das dritte Dornlumen und das vierte Dornlumen die distalen Enden des dritten Dorns (140A) und des vierten Dorns (150A) in der distalen Region (104) des Aspirationslumens (1101) leiten.

4. Katheter zur Entfernung von Blutgerinnseln nach Anspruch 2, wobei das erste Dornlumen (160) und das zweite Dornlumen (170) innerhalb einer Wand des Katheterkörpers (106) positioniert sind.

5. Katheter zur Entfernung von Blutgerinnseln nach Anspruch 4, wobei das erste Dornlumen (160) und das zweite Dornlumen (170) zwischen einer Außenhülle (106) und einer Innenauskleidung (170) des Katheterkörpers (106) positioniert sind.

6. Katheter zum Entfernen von Blutgerinnseln nach Anspruch 2, wobei das erste Dornlumen (160) und das zweite Dornlumen (170) relativ zum distalen Ende des Katheterkörpers (106) an unterschiedlichen Längspositionen zueinander angeordnet sind.

7. Katheter zum Entfernen von Blutgerinnseln nach Anspruch 1, wobei das erste Dornlumen (160) einen Winkel relativ zu einer Längsachse des Katheterkörpers (106) in einem Bereich von etwa 25 Grad bis 180 Grad aufweist.

8. Katheter zum Entfernen von Blutgerinnseln nach Anspruch 1, wobei der erste Dorn (140) ein Formgedächtnismaterial mit einer ihm verliehenen Gedächtnisform umfasst, um zu bewirken, dass ein distaler Abschnitt des ersten Dorns (140) radial nach innen in das Aspirationslumen (101) vorgespannt wird.

9. Katheter zur Entfernung von Blutgerinnseln nach Anspruch 1, ferner umfassend einen Griffabschnitt (200), der mit einem proximalen Ende (120) des Katheterkörpers (106) verbunden ist; wobei der Griff (200) einen Aktuator (250) umfasst, der mit dem ersten Dorn (140) verbunden ist; wobei der Aktuator (250) den ersten Dorn (140) translatorisch, rotatorisch oder beides bewegt.

10. Katheter zum Entfernen von Blutgerinnseln nach Anspruch 1, ferner umfassend einen Anschlag, der so konfiguriert ist, dass er verhindert, dass ein distales Ende (142) des ersten Dorns (140) distal aus dem Aspirationslumen (101) herausragt.

11. Katheter zum Entfernen von Blutgerinnseln nach Anspruch 1, wobei der distale Abschnitt (142) des ersten Dorns (140) die Form eines abgeflachten Rechtecks, eines Dreiecks oder eines Halbzylinders aufweist.

12. Katheter zum Entfernen von Blutgerinnseln nach Anspruch 1, wobei das erste Dornlumen (160) eine erste proximale Öffnung (164) innerhalb eines proximalen Abschnitts (120) des Katheterkörpers (106) beinhaltet.

13. Katheter zum Entfernen von Blutgerinnseln nach Anspruch 1, wobei die erste distale Öffnung (162) so geformt ist, dass der erste Dorn (140) in einem Winkel austritt, der im Allgemeinen parallel zur Längsachse des Katheterkörpers (106) oder in einem Bereich von etwa 5 Grad bis 90 Grad relativ zur Längsachse des Katheterkörpers (106) verläuft.

## Revendications

1. Cathéter d'extraction de caillots, comprenant :
un corps de cathéter (106) présentant une lumière d'aspiration (101) s'ouvrant à une extrémité distale (110) du corps de cathéter ; **caractérisé par** une première lumière de mandrin (160) s'étendant à travers le corps de cathéter et comportant une première ouverture distale (162) à l'intérieur d'une région distale de la lumière d'aspiration ; dans lequel la première lumière de mandrin (160) est positionnée à une périphérie du corps de cathéter ; un premier mandrin (140) situé à l'intérieur de la première lumière de mandrin ; dans lequel la première lumière de mandrin dirige une extrémité distale du premier mandrin (140) dans la région distale de la lumière d'aspiration afin de fatiguer un caillot qui est partiellement tiré dans la lumière d'aspiration.

2. Cathéter d'extraction de caillot selon la revendication 1, comprenant en outre une deuxième lumière de mandrin (170) positionnée à la périphérie du corps de cathéter (106) et comportant une seconde ouverture distale (172) à l'intérieur de la région distale (104) de la lumière d'aspiration (101) ; et un deuxième mandrin (150) situé à l'intérieur de la deuxième lumière de mandrin (170) ;
dans lequel la deuxième lumière de mandrin (170) dirige une extrémité distale (152) du deuxième mandrin (150) dans la région distale (104) de la lumière d'aspiration (101) afin de fatiguer le caillot (10) qui est partiellement tiré dans la lumière d'aspiration (101).

3. Cathéter d'extraction de caillot selon la revendication 2, comprenant en outre une troisième lumière de mandrin (160A) et une quatrième lumière de mandrin (170), toutes deux positionnées à la périphérie du corps de cathéter (106) et comportant des ouvertures dans la région distale (104) de la lumière d'aspiration (101) ; un troisième mandrin (140A) situé dans la troisième lumière de mandrin (160A) ; et, un quatrième mandrin (150A) situé dans la quatrième lumière de mandrin (170A) ; dans lequel la troisième lumière de mandrin et la quatrième lumière de mandrin dirigent les extrémités distales du troisième mandrin (140A) et du quatrième mandrin (150A) dans la région distale (104) de la lumière d'aspiration (1101).

4. Cathéter d'extraction de caillot selon la revendication 2, dans lequel la première lumière de mandrin (160) et la deuxième lumière de mandrin (170) sont positionnées à l'intérieur d'une paroi du corps de cathéter (106).

5. Cathéter d'extraction de caillot selon la revendication 4, dans lequel la première lumière de mandrin (160) et la deuxième lumière de mandrin (170) sont positionnées entre une enveloppe extérieure (106) et une chemise intérieure (170) du corps de cathéter (106).

6. Cathéter d'extraction de caillot selon la revendication 2, dans lequel la première lumière de mandrin (160) et la deuxième lumière de mandrin (170) sont positionnées à des positions longitudinales différentes l'une de l'autre, par rapport à une extrémité distale du corps de cathéter (106).

7. Cathéter d'extraction de caillot selon la revendication 1, dans lequel la première lumière de mandrin (160) a un angle par rapport à un axe longitudinal du corps de cathéter (106) dans une plage d'environ 25 degrés à 180 degrés.

8. Cathéter d'extraction de caillot selon la revendication 1, dans lequel le premier mandrin (140) comprend un matériau à mémoire de forme avec une forme mémorisée qui lui est conférée pour amener une partie distale du premier mandrin (140) à se déplacer radialement vers l'intérieur dans la lumière d'aspiration (101).

9. Cathéter d'extraction de caillot selon la revendication 1, comprenant en outre une partie poignée (200) connectée à une extrémité proximale (120) du corps de cathéter (106) ; la poignée (200) comprenant un actionneur (250) connecté au premier mandrin (140) ; dans lequel l'actionneur (250) déplace le premier mandrin (140) en translation, en rotation, ou les deux.

10. Cathéter d'extraction de caillot selon la revendication 1, comprenant en outre une butée conçue pour empêcher une extrémité distale (142) du premier mandrin (140) de sortir distalement de la lumière d'aspiration (101).

11. Cathéter d'extraction de caillot selon la revendication 1, dans lequel la partie distale (142) du premier mandrin (140) comprend la forme d'un rectangle aplati, d'un triangle ou d'un demi-cylindre.

12. Cathéter d'extraction de caillot selon la revendication 1, dans lequel la première lumière de mandrin (160) comprend une première ouverture proximale (164) à l'intérieur d'une partie proximale (120) du corps de cathéter (106).

13. Cathéter d'extraction de caillot selon la revendication 1, dans lequel la première ouverture distale (162) est formée de sorte que le premier mandrin (140) sort selon un angle généralement parallèle par rapport à un axe longitudinal du corps de cathéter (106) ou compris entre environ 5 degrés et 90 degrés par rapport à l'axe longitudinal du corps de cathéter (106).
